(19) **Europäisches Patentamt**

**European Patent Office**

**Office européen des brevets**

(11) **EP 1 271 351 A1**

(12) **EUROPEAN PATENT APPLICATION**
published in accordance with Art. 158(3) EPC

(43) Date of publication:
**02.01.2003 Bulletin 2003/01**

(51) Int Cl.⁷: **G06F 17/30**, C07K 7/06,
C07K 14/435, G01N 33/68

(21) Application number: **01912216.7**

(22) Date of filing: **09.03.2001**

(86) International application number:
**PCT/JP01/01846**

(87) International publication number:
**WO 01/067299 (13.09.2001 Gazette 2001/37)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **10.03.2000 JP 2000072485**

(71) Applicants:
• **DAIICHI PHARMACEUTICAL CO., LTD.**
Chuo-ku, Tokyo 103-8234 (JP)
• **FUJITSU LIMITED**
Kawasaki-shi, Kanagawa 211-8588 (JP)

(72) Inventors:
• **DOI, Hirofumi**
**Funabashi-shi, Chiba 273-0865 (JP)**
• **SUZUKI, Atsushi,**
**DAIICHI PHARMACEUTICAL CO., LTD**
**Edogawa-ku, Tokyo 134-8630 (JP)**

(74) Representative: **Schohe, Stefan**
**Forrester & Boehmert**
**Pettenkoferstrasse 20-22**
**80336 München (DE)**

(54) **METHOD OF ANTICIPATING INTERACTION BETWEEN PROTEINS**

(57) The present invention relates a method for predicting a protein or polypeptide (B) that interacts with a specific protein or polypeptide (A), wherein the method is characterized by comprising:

1) decomposing the amino acid sequence of protein or polypeptide (A) into a series of oligopeptides having a pre-determined length as sequence information;

2) searching, within a database of protein or polypeptide amino acid sequences, for a protein or polypeptide (C) comprising an amino acid sequence for each member of the series or for a protein or polypeptide (D) comprising an amino acid sequence homologous to an amino acid sequence for each member of the series;

3) carrying out local amino acid sequence alignment between said protein or polypeptide (A) and the detected protein or polypeptide (C) or detected protein or polypeptide (D); and

4) predicting whether the detected protein or polypeptide (C) and/or protein or polypeptide (D) is a protein or polypeptide (B) that interacts with the protein or polypeptide (A) based on the results of the local amino acid sequence alignment and a value calculated from a frequency of amino acids and/ or a frequency of said oligopeptides in said amino acid sequence database; and to a recording medi-um for carrying out the above method, a device comprising the recording medium, and proteins obtained thereby.

Fig.5

## SEQUENCE LISTING

<110> FUJITSU LIMITED

DAIICHI PHARMACEUTICAL CO., LTD.

<120> Method for predicting protein-protein interactions

<130> GP01-1001

<140> PCT/JP01/01846

<141> 2001-03-09

<150> JP P2000-72485

<151> 2000-03-10

<160> 1

<170> PatentIn Ver. 2.1

<210> 1

<211> 5

<212> PRT

<213> Homo sapiens

<400> 1

Asn Trp Gly Arg Ile
1               5

## Description

### FIELD OF THE INVENTION

The present invention relates to a prediction for protein-protein interactions, a method and a device therefor, and proteins obtained using the method and device.

### BACKGROUND OF THE INVENTION

Many proteins carry out their function by interacting with other proteins or the same protein. Thus, it is important to elucidate protein-protein interactions in the development of pharmaceuticals, the breeding in agriculture, and the like. Notably, along with the progress of genome analysis and cDNA analysis of various organisms including pathogenic microorganisms, the number of genes newly found and proteins encoded thereby whose functions are not known is rapidly increasing. Elucidating protein-protein interactions may permit one to predict the function of a protein whose function is not known.

A conventional method that has been used to screen for a protein interacting with a certain protein so as to elucidate their interactions, is the so-called two-hybrid system (Field, S. The two-hybrid system to detect protein-protein interaction. METHODS: A Companion to Meth. Enzymol., 5, 116-124, 1993). However, the two-hybrid system is a screening-based experiment, whose operation is complicated and time-consuming. Also, the number of proteins obtained is lower than that expected. In addition, this method has a disadvantage in that the results depend on the quality of the cDNA library used. In other words, this method has the risk that a gene encoding a protein interacting with a certain protein is not contained in the cDNA library used.

On the other hand, protein databases based on genome analysis and cDNA analysis have been enhanced, such that a method has also been adopted wherein a protein complex in a cell is subjected directly to MALDI-TOF mass spectrometry, followed by searching in the database for a fragment of the amino acid sequence thereof (Yates, JR 3rd, J. Mass Spectrom. 33, 1-19, 1998; Humphrey-Smith, I., et al., Electrophoresis, 18, 1217-1242; Kaufmann, R., 1995, J. Biotechnol., 41, 155-175, 1997). This method gives information concerning proteins that form a complex, but does not give any information concerning the protein-protein interaction. Thus, it must be experimentally confirmed which proteins interact with each other.

### SUMMARY OF THE INVENTION

In one embodiment, the present invention relates to a method for predicting a protein or polypeptide (B) that interacts with a specific protein or polypeptide (A), wherein the method is characterized by comprising:

1) decomposing the amino acid sequence of protein or polypeptide (A) into a series of oligopeptides having a pre-determined length as sequence information;
2) searching, within a database of protein or polypeptide amino acid sequences, for a protein or polypeptide (C) comprising an amino acid sequence for each member of the series or for a protein or polypeptide (D) comprising an amino acid sequence homologous to an amino acid sequence of each member of the series;
3) carrying out local amino acid sequence alignment between said protein or polypeptide (A) and the detected protein or polypeptide (C) or detected protein or polypeptide (D); and
4) predicting whether the detected protein or polypeptide (C) and/or protein or polypeptide (D) is a protein or polypeptide (B) that interacts with the protein or polypeptide (A) based on the results of the local amino acid sequence alignment and a value calculated from a frequency of an amino acid and/or a frequency of said oligopeptides in said amino acid sequence database.

One embodiment of the present invention may be the above-mentioned method for prediction wherein the oligopeptide is 4-15 amino acids in length.

In addition, in another embodiment, the present invention relates to a recording medium carrying a program to predict a protein or polypeptide (B) that interacts with a specific protein or polypeptide (A), comprising at least the following means a) to f):

a) a means for inputting amino acid sequence information of the protein or polypeptide (A) and storing the information;
b) a means for decomposing the above-mentioned information into a series of oligopeptides having a pre-determined length as sequence information, and a means for storing the sequence information consequently obtained;
c) a means for storing an input protein database;

d) a means for accessing the stored protein database and detecting a protein or polypeptide (C) having an amino acid sequence of said oligopeptide or a protein or polypeptide (D) having an amino acid sequence homologous to the amino acid sequence of said oligopeptide, and a means for storing and calculating a detected result;

e) a means for carrying out local alignment between the protein or polypeptide (A) and the detected protein or polypeptide (C) or protein or polypeptide (D), and a means for storing and calculating a result; and

f) a means for obtaining a resultant value of a frequency of an amino acid and/or a frequency of said oligopeptide from a protein database, followed by showing an index for predicting protein-protein interactions from the resultant value and a resultant value of said local alignment, and a means for storing and displaying the result and consequently detecting protein or polypeptide (B) which interacts with the protein or polypeptide (A).

In a further embodiment, the present invention relates to a recording medium comprising at least one of the following means g) to 1) in addition to the means a) to f):

g) a means for ranking strength of protein-protein interactions among detected proteins or polypeptides (B) based on the indexes calculated from a resultant value of local alignment and a resultant value of a frequency of an amino acid and/or a frequency of an oligopeptide in a protein database in the case that more than one protein or polypeptide (B) exist that are detected, and a means for storing and displaying the result;

h) a means for displaying full-length of amino acid sequences of the protein or polypeptide (A) and the protein or polypeptide (B) that is detected, followed by indicating a location of partial sequence to be aligned in the full-length sequence in the case that amino acid partial sequences are aligned by local alignment between the protein or polypeptide (A) and the protein or polypeptide (B);

i) a means for calculating a stereo structure model in the case that a stereo structure of the protein or polypeptide (A) or the protein or polypeptide (B) that is detected is known or in the case that homology modeling enable to make a stereo structure model, followed by displaying the structure of the amino acid partial sequences that are aligned by local alignment between the protein or polypeptide (A) and the protein or polypeptide (B) on the stereo structure;

j) a means of classifying proteins in a protein database to narrow a searching area and storing the same;

k) a means for serially inputting each protein in a protein database as the protein or polypeptide (A); and

l) a means for storing a genome database.

In still another embodiment, the present invention relates to a device for predicting protein-protein interactions comprising the means that are carried by the above-mentioned recording medium.

In an additional embodiment, the present invention relates to a method for specifying proteins or polypeptides that interact with each other, which comprises identifying a protein or polypeptide (B) that is predicted to interact with a specific protein or polypeptide (A) by the above-mentioned prediction method or prediction device, and then experimentally confirming the presence of the interaction between the protein or polypeptide (A) and the protein or polypeptide (B).

Furthermore, in another embodiment, the present invention relates to a protein or polypeptide that is specified by the above method.

In still another embodiment, the present invention relates to a method of screening for a compound that is capable of controlling the interaction of a specific protein or polypeptide (A) with a protein or polypeptide (B) utilizing the above-mentioned prediction method or prediction device.

In yet another embodiment, the present invention relates to a novel compound obtained by the screening method and a novel compound capable of controlling the interaction of the protein or polypeptide (A) with the protein or polypeptide (B) obtained by drug design based on information of the compound obtained.

In another embodiment, the present invention relates to an oligopeptide comprising amino acid sequence SEQ ID No: 1 which is capable of controlling the interaction of verotoxin 2 (VTII) with Bcl-2, or an oligopeptide that comprises an amino acid sequence homologous to the oligopeptide and is capable of controlling the interaction of VTII with Bcl-2, or a polypeptide that contains any of these oligopeptides and is capable of controlling the interaction of VTII with Bcl-2.

In addition, in one embodiment, the present invention relates to an agent against cell death comprising an oligopeptide comprising amino acid sequence SEQ ID NO: 1.

In still another embodiment, the present invention relates to a method of screening for a compound capable of controlling interaction of VTII with Bcl-2, wherein the method utilizes the above-mentioned oligopeptide and/or the above-mentioned polypeptide.

In yet another embodiment, the present invention relates to a method for determining a sequence of an oligonucleotide coding an oligopeptide involved in interaction of a specific protein or polypeptide (A) with a protein or polypeptide (B) that is predicted to interact with the protein or polypeptide (A), wherein the method uses the above-mentioned prediction method or the above-mentioned prediction device.

In a further embodiment, the present invention relates to a series of combinations of human proteins, which are predicted to interact with each other, identified by the above-mentioned prediction method or the above-mentioned prediction device.

In addition, in an embodiment, the present invention relates to a method for selecting a combination of proteins having a protein-protein interaction that is related to a disease, wherein the method comprises selecting the combination based on the information of a known protein that is related to the disease from the above-mentioned series of combination of proteins.

Further in another embodiment, the present invention relates to a series of combinations of proteins having protein-protein interaction that are related to diseases, and which are obtained by the above-mentioned method.

In yet another embodiment, the present invention relates to a method of screening for a compound that controls the interaction of a certain combination and/or two proteins further selected from the series of combinations of proteins having a protein-protein interaction that are related to diseases obtained as mentioned above.

In a still further embodiment, the present invention relates to a compound identified by the method of screening for a compound which controls the interaction.

In yet another embodiment, the present invention relates to a method for predicting a processing site of a protein by predicting the protein-protein interaction of a specific protein with an enzyme cleaving said protein using the above-mentioned prediction method or device.

In addition, in one embodiment, the present invention relates to an amino acid sequence that contains a protein-processing site obtained by the above-mentioned prediction method for a protein-processing site, and/or an amino acid sequence that contains a partial sequence homologous to the processing site.

## BRIEF DESCRIPTION OF DRAWINGS

Fig. 1a illustrates 20 amino acid residues from the amino terminal end of verotoxin 2.

Fig. 1b illustrates oligopeptides, each having an amino acid sequence length of 5 residues, which were obtained by decomposing the amino acid sequence consisting of said 20 residues as the sequence information using a program.

Fig. 1c illustrates oligopeptides, each having an amino acid sequence length of 6 residues, which were obtained by decomposing the amino acid sequence consisting of said 20 residues as the sequence information using a program.

Fig. 2 illustrates oligopeptides, each having an amino acid sequence length of 5 residues, which were obtained by decomposing the 13 residues from the amino terminal end of verotoxin 2 as the sequence information using a program, and human proteins comprising the amino acid sequence of the oligopeptides.

Fig. 3 illustrates the result of local alignment whereby oligopeptides that comprise a portion of verotoxin 2 (VTII) and human β-adrenergic receptor kinase 2 (ARK2) were obtained.

Fig. 4a illustrates the frequency of each amino acid in protein synthesis of *Escherichia coli.*

Fig. 4b illustrates the percentage that each amino acid is present in protein synthesis of *Escherichia coli.*

Fig. 5 is a simplified flow of means.

Fig. 6 illustrates amino acid sequences of oligopeptides derived from verotoxin 2 which are also present in human proteins that are related to cell death, and the corresponding human proteins.

Fig. 7 illustrates the result of local alignment whereby oligopeptides that comprise a portion of verotoxin 2 (VTII) and Bcl-2, were obtained.

Fig. 8 illustrates the result of local alignment whereby oligopeptides that comprise a portion of verotoxin 2 (VTII) and Bcl-xL, were obtained.

Fig. 9 illustrates the result of local alignment whereby oligopeptides that comprise a portion of verotoxin 2 (VTII) and MCL-1, were obtained.

Fig. 10a-b illustrate the result of local alignment whereby oligopeptides that comprise a portion of verotoxin 1 (VTI) and Bcl-2 (Fig. 10a) or Bcl-xL (Fig. 10b), were obtained.

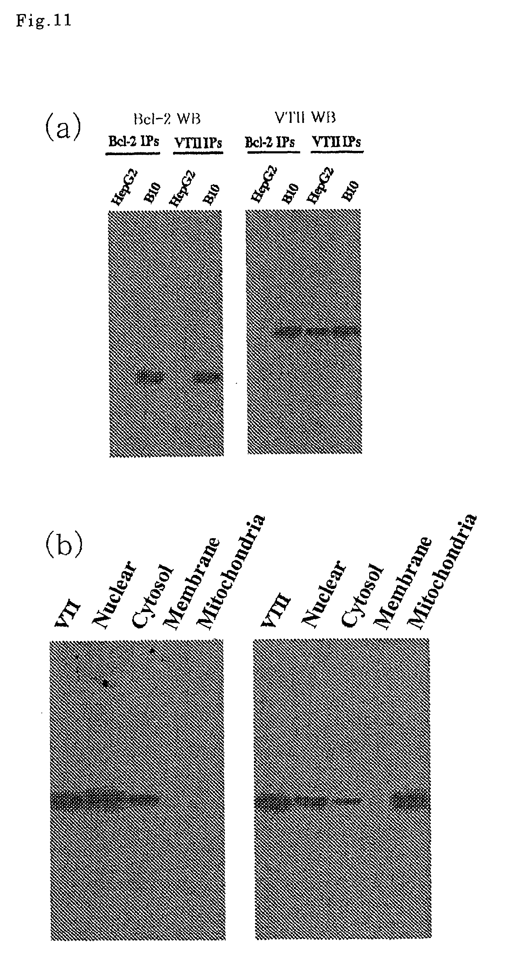

Figs. 11a-b illustrate electrophoretic patterns showing the result of confirmational experiments using HepG2 cells and B10 cells showing that verotoxin 2 (VTII) and Bcl-2 interact with each other ((Fig 11a) and (Fig 11b) right), but that verotoxin 1 (VTI) and Bcl-2 do not interact with each other ((Fig. 11b) left). In the figures, Bcl-2 IPs and VTII IPs indicate that Bcl-2 and VTII were immunoprecipitated by anti-Bcl-2 antibody and anti-VTII antibody, respectively. Fig. 11a (left) illustrates the result of western blotting using anti-Bcl-2 antibody (Bcl-2 WB). Fig. 11a (right) illustrates the result of western blotting using anti-VTII antibody (VTII WB). Fig. 11b illustrates electrophoretic patterns showing the result of confirmation of the subcellular fraction of B10 cells that were treated with verotoxin 1 (VTI) (left) or verotoxin 2 (VTII) (right), where these proteins were detected using anti-VTI antibody and anti-VTII antibody, respectively.

Fig. 12 illustrates the sites which correspond to the local alignment of verotoxin 2 (VTII) and Bcl-2.

Fig. 13 illustrates, using a wire model, the portion that is homologous to the partial sequence of verotoxin 2 (VTII) on the stereo structure of Bcl-xL.

Fig. 14 illustrates, using a wire model, the portion that is homologous to the partial amino acid sequence of Bcl-xL on the stereo structure of verotoxin 2 that is constructed by homology modeling.

Fig. 15 illustrates that oligopeptide NWGRI which comprises a portion of verotoxin 2 (VTII) and Bcl-2, suppresses cell death induced by VTII in a dose dependent manner of NWGRI.

Figs. 16a-b illustrate the result of local alignment whereby oligopeptides that comprise a portion of human helper T cell surface protein CD4 and HIV-1 virus surface protein gp120, were obtained. Fig. 16a illustrates oligopeptides that comprise a portion of CD4 and gp120. Fig. 16b illustrates amino acid sequences of a region having a high local homology in CD4 and gp120.

Fig. 17 illustrates the result of local alignment whereby oligopeptides that comprise a portion of CED-4 (a cell death-related protein of nematode) and MAC-1 protein (which binds to CED-4), were obtained.

Fig. 18 illustrates the result of local alignment whereby oligopeptides that comprise a portion of amyloid precursor protein (APP) and BASE (an enzyme which cleaves the protein), were obtained.

Figs. 19a-b illustrate the result of local alignment whereby oligopeptides that comprise a portion of furin-precursor protein (furin-pre) and von Willebrand factor precursor protein (VWF-pre), were obtained. Fig. 19a illustrates oligopeptides that comprise a portion of both proteins. Fig. 19b illustrates an amino acid sequence of a region having a high local homology in both proteins.

Fig. 20 illustrates the result of local alignment whereby oligopeptides that comprise a portion of amyloid precursor protein (APP) and protein PC7 (which is considered to be involved in the processing thereof), were obtained. In the figure, symbol "=" indicates a site that is predicted to be a cleavage site.

## BRIEF DESCRIPTION OF REFERENCE NUMERALS

a Means for inputting
b Means for decomposing into a series of oligopeptides and storing the same
c Means for storing
d Means for searching and storing
e Means for carrying out local alignment and storing
f Frequency-calculating/memory-displaying means
g Ranking/memory-displaying means
h Location-displaying means
i Stereo structure-calculating/memory-displaying means
j Means for classifying proteins and storing
k Sequentially inputting means
l Means for storing
m Keyboard
n Controlling means
o Outputting means

## DETAILED DESCRIPTION OF THE INVENTION

The embodiments of the present invention will be described in more detail below as well as the principle and method of the present invention, a recording medium that carries a program for carrying out the method, a device that works the function, proteins and polypeptides that are obtained by the method and device. The following description is given only for illustration, and it not intended to limit the present invention.

Technical and scientific terms used in the specification have the meanings usually understood by one of ordinary skill in the art to which the present invention pertains, unless otherwise defined. Reference is made herein to various methodologies known to those of ordinary skill in the art. Publications and other materials setting forth such known methodologies to which reference is made are incorporated herein by reference in their entireties.

In one embodiment, the present invention relates to a method for predicting protein-protein interactions, which is based on the following idea: protein is composed of a sequence consisting of 20 kinds of amino acids, but these amino acids are not randomly placed. Therefore, it is considered that an oligopeptide that is a partial sequence of a protein has a role in a species of a living organism.

For example, an oligopeptide that is a part of a certain enzyme is considered to play a role in recognizing a substrate. In another protein, an oligopeptide that plays an important role in interacting with other proteins is considered to exist. In this way, it is necessary to consider the function or interaction of a protein from the oligopeptide level. In addition, from the viewpoint of frequency of the oligopeptide, the frequency of the appearance of certain oligopeptides in all of the proteins encoded by the genome in one organism is not even. Some oligopeptides frequently occur in various proteins; others do so only rarely. It is very likely that an oligopeptide that occurs with low frequency is an oligopeptide that is unique to each protein. Such an oligopeptide might determine the feature or function of the protein.

On the other hand, the fact that proteins interact with each other implies that the interacting proteins perform a function in cooperation with each other whereby the organism carries on its biotical activity. If it is assumed that one oligopeptide corresponds to one function, two proteins that interact with each other might have the same oligopeptide or homologous oligopeptides. In addition, these two proteins might have homologous sequence structure in a part other than the oligopeptide that is the same.

As one of the techniques of similarity search for analyzing homology of two proteins, a method of comparison by aligning the primary structures of both proteins is known (Minoru Kanahisa "Introduction to genome informatics (in Japanese)" Kyoritsu Publishing Co., Ltd., 93-104, 1996). This sequence alignment includes 'global alignment' and 'local alignment'. The 'global alignment' comprises aligning the entire sequences, and the 'local alignment' comprises locally aligning only homologous parts extracted from their sequences. In any alignment, the alignment is carried out so that the relation between/among two sequences or more can be as clear as possible. Many combinations exist in the alignment depending on the length of the sequence. Methods for carrying out combinatorial optimization include the dynamic programming method. The Smith-Waterman method (Smith, TF and Waterman, MS, J. Mol. Biol. 147, 195-197, 1981) that is based on the principle that dynamic programming gives an estimation function on the combinatorial optimization of sequences. A value of the estimation function, i.e., 'homology score' or 'score' permits estimating homology between two proteins. It can be estimated that the higher the score between proteins that were compared, the higher the homology between these proteins. As for the local alignment, it is carried out by setting a threshold to the score, followed by carrying out combinatorial optimization of partial sequences, when the combination of sequences is searched by dynamic programming (e.g., Gotoh, O., Pattern matching of biological sequences with limited storage, Comput. Appl. Biosci. 3, 17-20, 1987). The local alignment method permits searching the homologous structure in a part of the protein other than where the oligopeptide that is the same portion to two proteins is located.

Protein-protein interactions might have been conserved in the process of evolution. The case of verotoxin of *Escherichia coli* and Bcl-2, as described later, implies that one function has been conserved in the protein-protein interaction beyond species, whence structurally similar amino acid sequences might exist. In addition, in the processing of amyloid precursor protein and von Willebrand Factor (VWF) precursor protein, as described later, the function of proteolysis might have been conserved in the protein-protein interaction, and structurally similar amino acid sequences might exist.

A method for predicting protein-protein interactions that is created based on the above idea may also permit predicting a network of functions that has been known only as a single function in the past and describing a new image of life based on the results that were predicted on a computer and on the overall relation of actions that is different from the image of life being reached by the accumulation of facts that have been obtained by the enumeration principle of molecular biology.

In addition, if the prediction for interactions is possible not in one organism but between two organisms, e.g., human beings and pathogenic microorganisms, the elucidation of the pathogenesis that has not been known so far might become possible.

Concretely, one embodiment of the above method for predicting protein-protein interactions is a method of extracting and predicting a counter-protein from a protein database and the like, which interacts with a protein that was obtained by genome analysis or cDNA analysis whose function is unknown or a protein whose function is known, wherein the method comprises, for example, the following steps 1-4:

In step 1, an amino acid sequence of a specific protein or polypeptide is decomposed into a series of oligopeptides having a pre-determined length as the sequence information. In step 2, proteins or polypeptides are determined which comprise each oligopeptide. In step 3, homology of partial structures between the proteins is estimated by local alignment. In step 4, each oligopeptide is further evaluated by a frequency of occurrence.

Each step will be described below more in detail:

Step 1:

The amino acid sequence of a specific protein or polypeptide (A), such as a protein or polypeptide that is obtained by genome analysis or cDNA analysis and whose function is not known or a protein or polypeptide whose function is known, is decomposed into oligopeptides as sequence information by shifting, serially, by one amino acid residue from the amino terminal end to the carboxyl end.

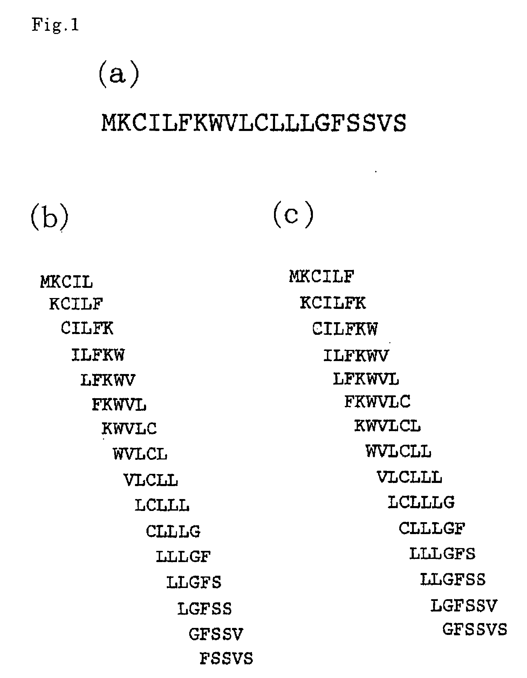

For example, Figs. 1a-b illustrate oligopeptides (Fig. 1b) having an amino acid length of 5 residues that were obtained by decomposing the first 20 residues (Fig. 1a) of verotoxin 2 (VTII) of *Escherichia coli* O157:H7 from its amino terminal end as sequence information. Amino acids and oligopeptides are given in their one-letter symbols hereafter.

When step 1 is carried out, the amino acid length of oligopeptide that is decomposed as the sequence information is 4 to 15 residues, preferably 4 to 8 residues. The longer the length of an oligopeptide, the greater the particularity of the oligopeptide, as shown in Example 2.

Step 2:

In step 2, a protein or polypeptide (C) comprising an amino acid sequence of an oligopeptide that was obtained by the decomposition in step 1 or a protein or polypeptide (D) having an amino acid sequence that is homologous to the oligopeptide is searched for in an amino acid sequence database of proteins or polypeptides. The number of detected proteins or polypeptides (C) or (D) can be large or can be one depending on the oligopeptide used.

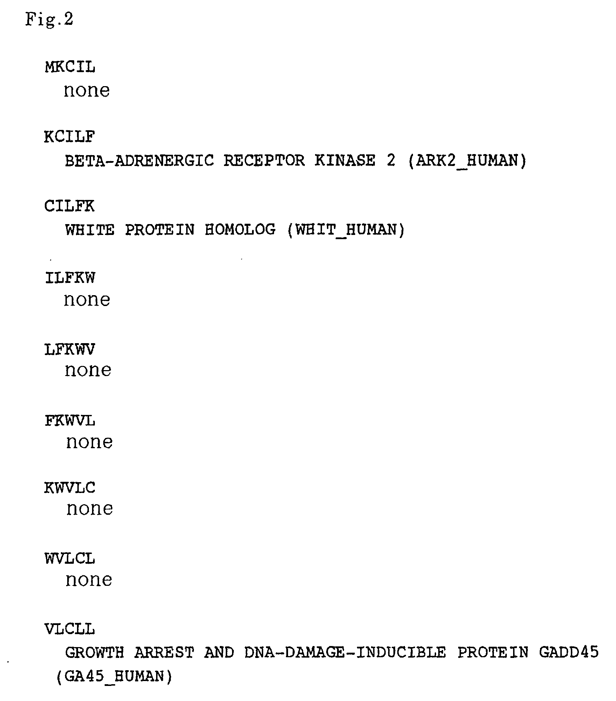

For example, Fig. 2 illustrates the results of searching for proteins having 9 oligopeptides each consisting of 5 amino acids obtained by decomposing 13 residues of verotoxin 2 (VTII) of *Escherichia coli* O157:H7 from the amino terminal end, in a protein database (SWISS-PROT version 35). Verotoxin 2 causes food poisoning and/or renal damage in human beings, so that human protein can be targets for searching for counter-proteins that interact with VTII. For example, such a search shows that a human protein comprising oligopeptide KCILF shown in Fig. 2 (second) is β-adrenergic receptor kinase 2 (ARK2 HUMAN).

Step 3:

Local alignment is carried out between the above protein or polypeptide (A) and the protein or polypeptide (C) or protein or polypeptide (D) that is obtained in the search in Step 2.

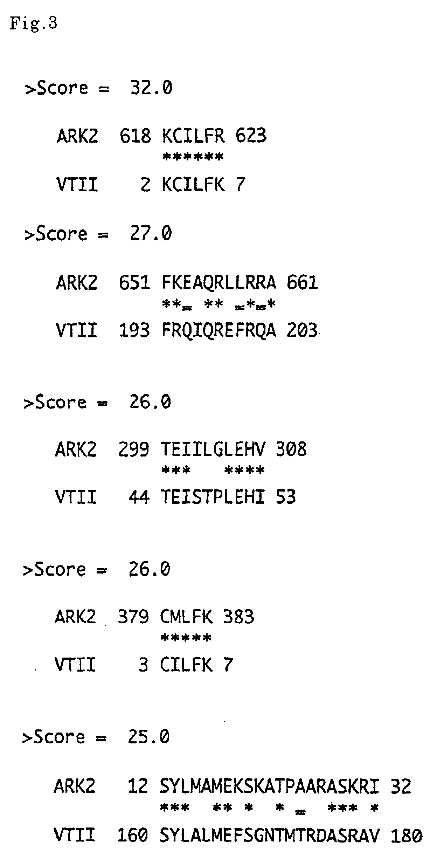

For example, Fig. 3 illustrates the result of local alignment between verotoxin 2 (VTII) and β-adrenergic receptor kinase 2 (ARK2).

Step 4:

If the result of the local alignment in step 3 shows any homology of partial sequence between the above protein or polypeptide (A) and the detected protein or polypeptide (C) and/or protein or polypeptide (D), the protein or polypeptide (C) and/or protein or polypeptide (D) are/is predicted to possibly be a protein or polypeptide (B) that interacts with protein or polypeptide (A). Moreover, the frequency of amino acid(s) and/or the frequency of oligopeptide that is present in both protein or polypeptide (A) and the detected protein or polypeptide (C) and/orproteinorpolypeptide (D) is calculated from a protein database, followed by evaluating the particularity of each oligopeptide in the protein database or in the genome of an organism having the protein or polypeptide (A) or the detected protein or polypeptide (C) and/or protein or polypeptide (D). If the particularity is high, the reliability of the prediction is evaluated to be high that the protein or polypeptide (C) and/or protein or polypeptide (D) are/is a protein or polypeptide (B) that interacts with the protein or polypeptide (A).

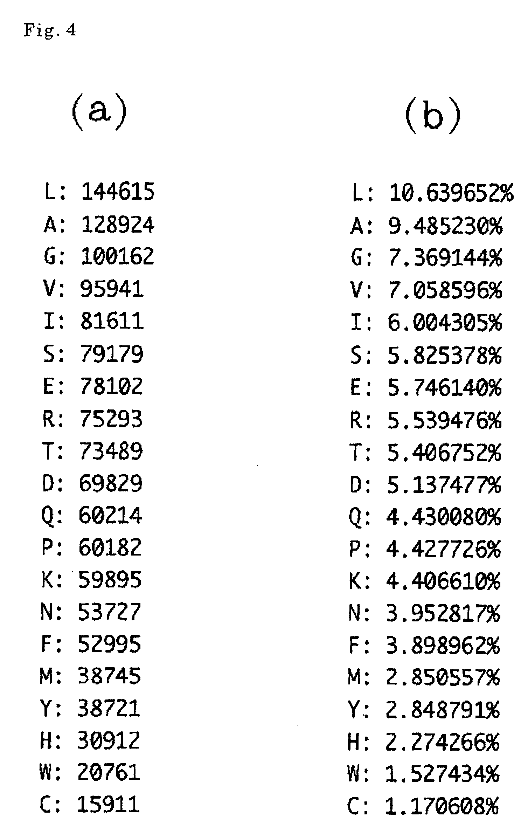

For example, an index of particularity of the above oligopeptide KCILF is calculated to be $1284.86 \times 10^{-10}$ from the composition ratio (Fig. 4b) that is calculated from the frequency of amino acid (Fig. 4a) in all of the proteins encoded by the genome of *Escherichia coli* shown in Fig. 4, so that the particularity is high. An oligopeptide consisting of 5 amino acids that is calculated to have low particularity in the *E. coli* genome is LLLLL, i.e., the particularity index is $136344.34 \times 10^{-10}$. An oligopeptide consisting of 5 amino acids that is calculated to have the highest particularity is CCCCC, with a particularity index of $2.208 \times 10^{-10}$, but the oligopeptide is not found in the *E. coli* genome. Therefore, the prediction that verotoxin 2 interacts with β-adrenergic receptor kinase 2 is evaluated to have high reliability from the value of the particularity index.

In order to confirm further the protein-protein interaction, the gene encoding the protein may be cloned for expression based on the information of the obtained proteins that interact with each other. For example, as described in the examples later, VTII and Bcl-2 were predicted to interact with each other by the above predicting method or a predicting device carrying a program for the predicting method. This could be confirmed by experiments wherein cells in which Bcl-2 is expressed and cells in which Bcl-2 is not expressed were treated with VTII, followed by co-immunoprecipitating with anti-Bcl-2 antibody and anti-VTII antibody. In addition, it is possible to specify the oligopeptide as an important interacting site, for example, by introducing a mutation by a well-known method into the amino acid sequence of an oligopeptide that is predicted to be an interacting site, followed by confirming that the interaction is lost. The method for confirming interactions experimentally is not limited to the above ones, but any of techniques that are applicable by those skilled in the art may be used.

In addition, if it is confirmed that an oligopeptide, which was predicted to be an interacting site, interrupts a protein-protein interaction and suppresses any function or action of the protein, then such an oligopeptide can be utilized as an agent for suppressing the action of the protein. For example, as described in an example below, oligopeptide NWGRI, which was predicted to be the interacting site for VTII and Bcl-2, suppresses cell death induced by VTII and can be used as an agent against cell death. Such a low-molecular-weight compound can be utilized as pharmaceuticals, reagents, and the like.

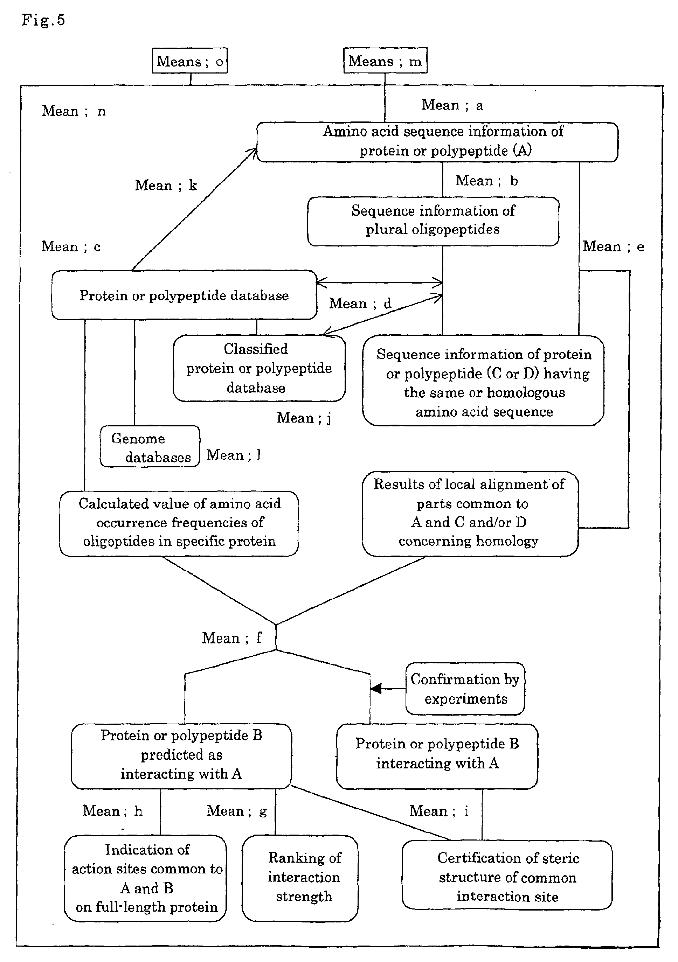

Next, a recording medium and device that carry a program for the above method for predicting protein-protein interactions will be described. The above recording medium and device comprise at least the following means (a) to (f) . Fig. 5 illustrates an example of the constitution.

Inputting means (a):

A means for inputting the amino acid sequence information concerning a specific protein or polypeptide (A) such as a protein or polypeptide, which was obtained by genome analysis or cDNA analysis, whose function is not known or protein or polypeptide whose function is known. Means for decomposing into a series of oligopeptides and storing the same (b):

A means for decomposing the amino acid sequence information that was input by inputting means (a) into a series of oligopeptides having a pre-determined length as sequence information by shifting, serially, by one amino acid residue from the amino terminal end to the carboxyl end, and storing the result.

Storing means (c):

A means for storing a database that was input in concerning with a protein or polypeptide.

Searching/storing means (d):

A means for accessing a database concerning a protein or polypeptide that is stored in storing means (c), followed by searching for a protein or polypeptide (C) comprising the amino acid sequence of the above oligopeptide or a protein or polypeptide (D) comprising an amino acid sequence that is homologous to the amino acid sequence of the above oligopeptide, and storing the result.

Carrying out local alignment/storing means (e):

A means for carrying out local alignment between the above protein or polypeptide (A) and the detected protein or polypeptide (C) and/or (D), and storing the result.

Frequency-calculating/memory-displaying means (f):

A means for calculating an index for predicting protein-protein interactions from the result of the above local alignment and the result obtained after calculating a frequency of an amino acid and/or a frequency of an oligopeptide in a peptide or polypeptide database, and storing and displaying the result.

In addition, in the above program for predicting protein-protein interaction, it is also possible to comprise the following means (g) to (l) in an appropriate combination:

Ranking/memory-displaying means (g):

A means having a function of ranking proteins or polypeptides (B), when more than one protein or polypeptide (B) is detected, by using the result of the local alignment and the result of the calculation of a frequency of an amino acid and/or a frequency of an oligopeptide from a protein database as indexes and a function of storing/displaying the result.

Location-indicating means (h):

A means for displaying full-length amino acid sequences of the protein or polypeptide (A) and the protein or polypeptide (B) followed by indicating a location of partial sequence to be aligned in the full-length sequences in the case that amino acid partial sequences are aligned between the protein or polypeptide (A) and the detected protein or polypeptide (B).

Stereo structure-calculating/memory-displaying means (i):

A means for calculating a stereo structure model followed by displaying the structure of the amino acid partial sequences that are aligned between the protein or polypeptide (A) and the protein or polypeptide (B) on the stereo structure in the case that a stereo structure of the protein or polypeptide (A) or the protein or polypeptide (B) that is detected is known or in the case that a stereo structure model can be constructed by homology modeling.

Protein-classifying/storing means (j):

A means having a function of classifying proteins or polypeptides in a protein or polypeptide database by feature, function, and/or origin to narrow a searching area followed by storing them.

Sequentially inputting means (k):

A means for sequentially inputting each protein or polypeptide in a protein or polypeptide database as the protein or polypeptide (A).

Storing means (l):

A means having a function of storing a genome database.
The above means are carried on an appropriate medium.
As one embodiment of use, each of these means may be provided as a device containing a recording medium selectively carrying it as a program. A device for predicting protein-protein interactions is operated as described below (see Fig. 5).
A specific protein or polypeptide (A), such as a protein or peptide which was input by an inputting means (a) and whose function is unknown or known, is decomposed by means for decomposing into a series of oligopeptides and storing the same (b) into a series of oligopeptides having a pre-determined length as sequence information, and the oligopeptides are stored. In this case, the protein or polypeptide (A) is sequentially input by a sequentially inputting means (k) from a means (c) that stores a database that was input concerning the protein or polypeptide, when desired. A search is carried out through a means (c) for the amino acid sequence of the above-stored oligopeptide by a searching/storing means (d), and a protein or polypeptide (C) comprising the amino acid sequence of the oligopeptide or a protein or polypeptide (D) comprising an amino acid sequence that is homologous to the amino acid sequence of the oligopeptide is detected and stored. When searching, it is also possible to classify proteins or polypeptides in a database to narrow the searching area by a protein-classifying/storing means (j), followed by searching within the resultant area. The above protein or polypeptide (A) and the detected protein or polypeptide (C) or (D) are subjected to local alignment by a locally aligning/memory-displaying means (e), and the result is stored. Next, by a frequency-calculating/storing means (f), the frequency of an amino acid and/or the frequency of the oligopeptide are/is calculated from a database that was stored on a means (c), and an index for predicting protein-protein interactions is calculated from the result and the above-obtained result of the local alignment and stored. Then, those obtained are displayed on the screen of the device, which are the protein or polypeptide (C) or (D) that are predicted to interact with the above protein or polypeptide (A), an amino acid sequence of an oligopeptide that is the same in these proteins, a frequency of the oligopeptide, indexes for predicting protein-protein interactions, and so on. Displayed results permits giving a protein or polypeptide (B) that has interaction with the above protein or polypeptide (A) based on the indexes for predicting protein-protein interactions. In addition, concerning the above protein or polypeptide (B), it is also possible to display the functional information of a protein that is stored on a means (c) and the gene information from a means (l) equipped when desired that stores a genome database. When more than one protein or polypeptide (B) is detected, a ranking/ memory-displaying means (g) permits ranking the protein or polypeptide (B) in order of the particularity to interact with the above protein or polypeptide (A). It is also possible to indicate by a location-indicating means (h) which part of the full-length amino acid sequences of the above protein or polypeptide (A) and the detected protein or polypeptide (B) is the partial amino acid sequence that is aligned between the protein or polypeptide (A) and the protein or polypeptide (B). In addition, it is also possible to display a stereo structure of the protein or polypeptide (A) and the protein or polypeptide (B), as well as the part that is aligned between the protein or polypeptide (A) and the protein or polypeptide (B) by a stereo structure-calculating/memory-displaying means (i). This device may be equipped with keyboard (m) , controlling means (n), outputting means (o), as shown also in Fig. 5, and so on as well as these means (a) - (1).
The above method for predicting protein-protein interactions or the above prediction device can further be used for screening for a novel compound that controls the interaction of a specific protein or polypeptide (A) with a protein or polypeptide (B). The above method of screening for a novel compound that controls the interaction of a specific protein or polypeptide (A) with a protein or polypeptide (B) is carried out based on the information of the amino acid sequence of a key oligopeptide. An amino acid sequence of a selected oligopeptide, an amino acid sequence of an oligopeptide homologous thereto, or a polypeptide comprising the amino acid sequence or the homologous amino acid sequence per se can be capable of controlling the interaction of the protein or polypeptide (A) with the protein or polypeptide (B). For example, in the case that the protein or polypeptide (B) having a receptor function to the protein or polypeptide (A) is in existence, it is likely that an oligopeptide that is screened by the above technique is antagonistic to the interaction of the protein or polypeptide (A) with the protein or polypeptide (B). For example, in the case that the protein or polypeptide (A) is activated by the interaction with the protein or polypeptide (B), it is likely that an oligopeptide that is screened by the above technique has a function as an agonist.

Concretely, as described in detail in the examples below, it was experimentally confirmed that an oligopeptide NWGRI described in SEQ ID NO: 1, which comprises a portion of VTII and Bcl-2, that were predicted and experimentally confirmed to interact with each other by the present invention, interrupts complex formation due to the interaction of VTII with Bcl-2, and suppresses cell death induced by VTII. Therefore, NWGRI oligopeptide can be used as a medicament for controlling a disease related to cell death induced by VTII, for example as a medicament for treating a disease caused by *Escherichia coli* 0157 expressing VTII, more concretely as an agent against cell death. Moreover, an oligopeptide having an amino acid sequence homologous to the amino acid sequence and capable of controlling the interaction of VTII with Bcl-2, or a polypeptide comprising the amino acid sequence or an amino acid sequence homologous to the amino acid sequence and capable of controlling the interaction of VTII with Bcl-2, can also be used as a medicament for controlling a disease related to cell death induced by VTII. In addition, a novel compound capable of controlling the interaction of VTII with Bcl-2 can be obtained utilizing these oligopeptides and polypeptides by the drug design method or by applying of a known screening method.

In this way, a novel compound, which is obtained by drug design based on the information of an oligopeptide that is obtained by the above screening method according to an embodiment of the present invention, is capable of controlling the interaction of a specific protein or polypeptide (A) with a protein or polypeptide (B). Namely, to predict interaction of the above protein or polypeptide (A) with the above protein or polypeptide (B) permits one to make a derivative of the oligopeptide obtained by the above screening method and a low-molecular-weight compound having a structure homologous to the oligopeptide by a well-known drug design technique.

The above prediction method is also very useful for a method for determining the sequence of the oligonucleotide coding an oligopeptide involved in interaction of a specific protein or polypeptide (A) with a protein or polypeptide (B). Applying well-known methods such as substitution, deletion, addition, insertion, or induced mutation based on this information permits one to obtain a useful oligonucleotide. The obtained oligonucleotide can be used for obtaining a compound for controlling the interaction of protein or polypeptide (A) with protein or polypeptide (B) on a gene level. For example, it is utilized for making an antisense oligonucleotide to interrupt the protein-protein interaction. In addition, the obtained oligonucleotide can be used for diagnosing a disease that is related to the protein-protein interaction.

In another embodiment, the present invention relates to a series of combinations of human proteins that are predicted to have protein-protein interactions, which are predicted by the above method or device for predicting protein-protein interaction. The series of combination of proteins can be provided as a catalogue or as a database. A series of combination of proteins which interact with each other that are involved in a disease can be obtained by selecting ones having protein-protein interactions that are related to a disease based on the information of known proteins that can be related to the disease from the series of combination of proteins having protein-protein interactions. These can be provided as a catalogue or as a database. These combinations of proteins are useful as a medicament for treating or preventing diseases or as ways to obtain medicaments. For example, a compound capable of controlling interaction of two proteins can be obtained by screening using a well-known screening method and by utilizing a combination of proteins that is obtained.

In the case that among combinations consisting of two proteins having a protein-protein interaction, one protein is an enzyme capable of processing protein and cleaves the other protein, the processing site of the protein that is cleaved can be predicted by the above method or device for predicting protein-protein interactions.

For example, as shown in an example below, prediction could be accomplished on the subject of the interaction of an amyloid precursor protein with an enzyme that is involved in its processing, and on the subject of the interaction of von Willebrand factor precursor protein with an enzyme furin that is involved in its processing. Namely, the above method or device for predicting protein-protein interactions permits predicting a cleavage site when a precursor protein is cleaved to act as a mature protein. In this way, a hitherto-unknown enzyme having a protein-processing action related to a disease and a protein that is cleaved by the enzyme can be obtained by predicting protein-protein interactions.

## Examples

Although advantages, features, and possible applications of the present invention are described below in greater detail with reference to exemplary embodiments, the present invention is not limited to the following examples. In addition, although SWISS-PROT version 35 was used as a protein database in the following examples, other protein databases or the like can also be used.

## Example 1

Figs. 1a-c illustrate oligopeptides that were decomposed from the first 20 residues (Fig. 1a) of verotoxin 2 (VTII) of *Escherichia coli* O157:H7 from the amino terminal end, where the oligopeptides have an amino acid sequence length of 5 resides (Fig. 1b) as an example of step 1. Fig. 1c illustrates oligopeptides that were decomposed from the first 20 residues of verotoxin 2 (VTII) from the amino terminal end, where the oligopeptides have an amino acid sequence length of 6 residues.

## Example 2

In step 4 of the above method for predicting protein-protein interactions, values are used as an index for predicting the interaction of proteins or polypeptides. The values are calculated from the frequency of an amino acid in a protein or polypeptide database and the frequency of an oligopeptide in the protein or polypeptide database. By way of example, the particularity of oligopeptides is calculated from the frequency of the amino acid in all of the proteins encoded by the genome of *Escherichia coli* shown in Figs. 4a-b. The percentage 'Ai' of each amino acid 'ai' can be calculated to be as shown in Fig. 4b from the frequency of occurrence (Fig. 4a) of the 20 kinds of amino acids in all of the proteins encoded by the genome of *Escherichia coli.*

The particularity of oligopeptide ala2a3a4a5 is calculated to be A1×A2×A3×A4×A5. For example, in the case of oligopeptide KCILF, it is calculated to be $4.406610 \times 1.170608 \times 6.004305 \times 10.639652 \times 3.898962 \times 10^{-10}$. The particularity of oligopeptide LLLLL is calculated to be $136344.34 \times 10^{-10}$, and the particularity of oligopeptide CCCCC is calculated to be $2.20 \times 10^{-10}$.

The smaller the value, the greater the particularity of the oligopeptide. The oligopeptide that has the highest particularity among those having an amino acid sequence length of 5 residues is oligopeptide CCCCC, but this oligopeptide does not occur in any of the proteins encoded by the genome of *Escherichia coli.* In contrast, the oligopeptide that has the lowest particularity is oligopeptide LLLLL.

When a protein or polypeptide (A) is decomposed into oligopeptides in step 1, the longer the oligopeptide, the greater the particularity of the oligopeptide.

## Example 3

In step 4 of the above method for predicting protein-protein interactions, as an index for predicting the interaction of proteins or polypeptides, the result of the local alignment is used. Here is mentioned an example in which scores of the alignment of a partial sequence by Gotoh's method (Gotoh, O., Pattern matching of biological sequences with limited storage, Comput. Appl. Biosci. 3, 17-20, 1987) are used. In the following examples, when the score was 25.0 or higher, it was judged that the partial amino acid sequences are aligned (homologous) between a protein or polypeptide (A) and another protein or polypeptide (B).

The "m" amino acid partial sequences are premised to be aligned between a protein or polypeptide (A) and another protein or polypeptide (B) with their scores being Si ($1 \leqq i \leqq m$) and the amino acid length of protein or polypeptide (B) being LB. The index for predicting the interaction of protein or polypeptide (A) with protein or polypeptide (B) calculated from the result of local alignment is defined as the sum (Si)/LB. It is predicted that the higher the index, the stronger the interaction.

## Example 4

### Prediction of interaction of VTII with Bcl-2

Verotoxin 2 (VTII) of *Escherichia coli* O157:H7 causes food poisoning and renal damage in human beings, but the mechanism of action is not well-known (Sandvig, K., et al., Exp. Med. Biol. 412, 225-232, 1997; Paton, JC., and Paton, AW. Clin. Microbiol. Rev. 11, 450-479, 1998). This protein is a toxic protein. Therefore, human proteins relating to cell death serve as candidates of proteins interacting with this protein. Thus, human proteins that may interact with this protein were searched, specifically for human proteins relating to cell death, in protein database SWISS-PROT version 35, and an example is given below showing that they actually interact with each other.

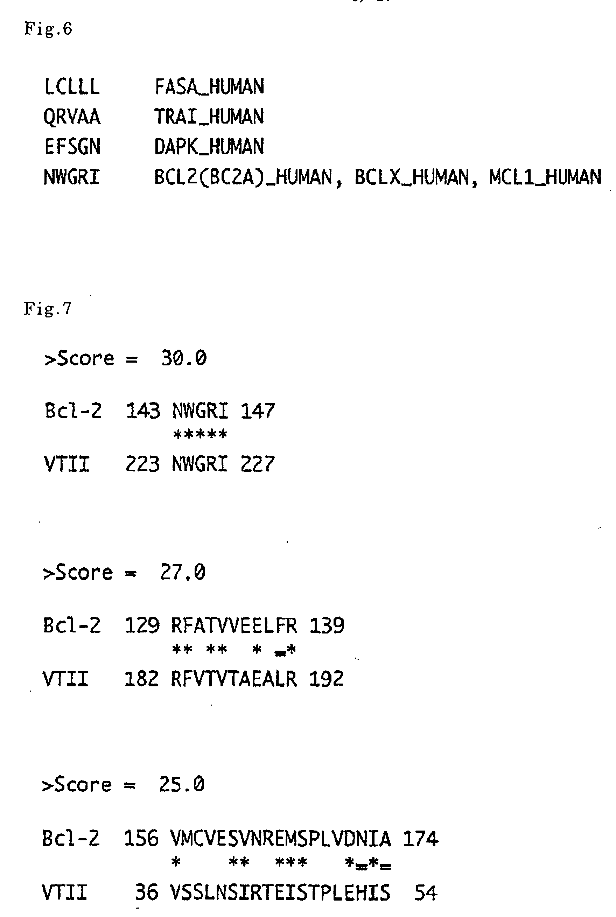

Among oligopeptides having an amino acid sequence length of 5 residues of verotoxin 2, those found to be contained in a human protein relating to cell death were the following four, i.e., LCLLL, QRVAA, EFSGN, and NWGRI, in SWISS-PROT version 35 (see Fig. 6, where the human proteins are shown by using protein IDs of SWISS-PROT version 35). Values of particularity for these oligopeptides were calculated from the amino acid frequencies in all of the proteins encoded by the genome of *Escherichia coil* shown in Figs. 4a-b, i.e., the particularity of LCLLL was $15001.03 \times 10^{-10}$; that of QRVAA was $15584.55 \times 10^{-10}$; that of EFSGN was $3801.65 \times 10^{-10}$; that of NWGRI was

$1479.85 \times 10^{-10}$. It was found that NWGRI has the highest particularity among these four oligopeptides.

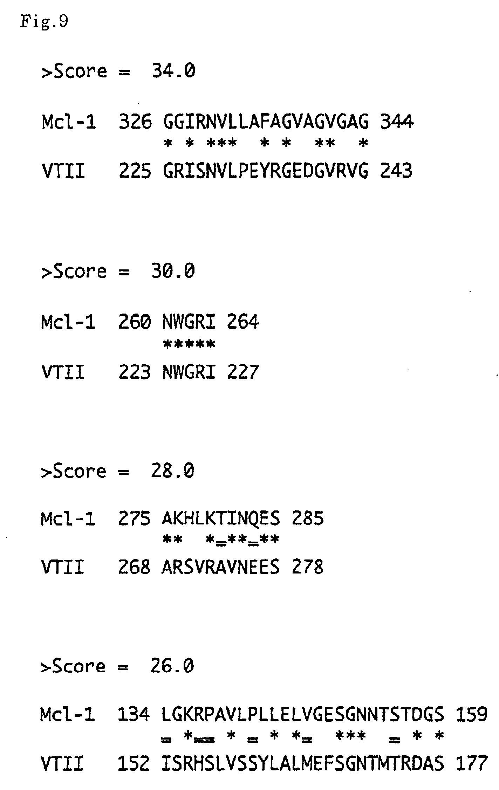

Oligopeptide NWGRI comprises a portion of verotoxin 2 and each of three human proteins, i.e., Bcl-2, Bcl-xL, and MCL-1. Local alignment between verotoxin 2 (VTII) and each of Bcl-2, Bcl-xL, and MCL-1 revealed partial homology in their amino acid sequence, as shown in Figs. 7, 8, and 9. Then, the sum of the scores of the local alignment was divided by the length of each protein to give index as described in Example 3, and shown below.

$$Bcl\text{-}2 \ (30.0 + 27.0 + 25.0) / 239 = 0.343$$

$$Bcl\text{-}xL \ (30.0 + 29.0 + 27.0) / 233 = 0.369$$

$$MCL\text{-}1 \ (34.0 + 30.0 + 28.0 + 26.0) / 350 = 0.337$$

Among these three proteins, Bcl-2 and Bcl-xL constitutes the same family. Based on the index calculated from the local alignment by the above method, the prediction is that Bcl-2 and Bcl-xL have higher interaction with verotoxin 2 among Bcl-2, Bcl-xL and MCL-1.

Verotoxin 1 (VTI) is one of the verotoxins produced by *Escherichia coli* O157:H7, and is an isoform of verotoxin 2. The toxicity of verotoxin 1 is weaker than that of verotoxin 2, with the former being about one fiftieth the latter (Tesh, VL., et al., 1993, Infect. Immun. 61, 3392-3402). In protein database SWISS-PROT version 35, a human protein that contains an oligopeptide having an amino acid length of 5 residues that comprises a portion of verotoxin 1 and is related to cell death is P2X1_HUMAN, the oligopeptide being SSTLG. However, the particularity of oligopeptide SSTLG, which is calculated to be $14385.63 \times 10^{-10}$ from the amino acid frequencies in all of the proteins encoded by the genome of *Escherichia coli* shown in Figs. 4a-b, is lower than that of NWGRI, by about one tenth.

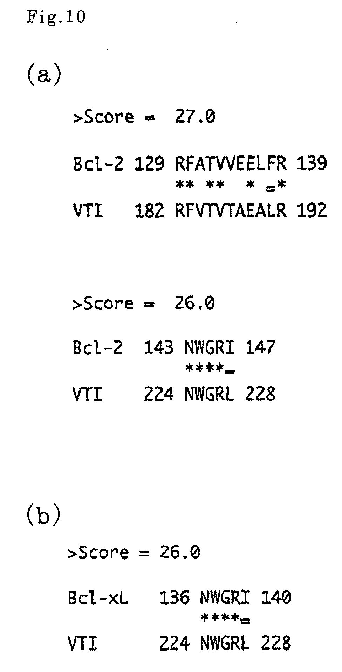

In verotoxin 1, the oligopeptide NWGRL that corresponds to oligopeptide NWGRI having an amino acid length of 5 residues in verotoxin 2 reveals a particularity of $2622.30 \times 10^{-10}$, calculated from Figs. 4a-b, that is lower than that of NWGRI. Both Bcl-2 and Bcl-xL contain oligopeptide NWGR having an amino acid sequence length of 4 residues that comprises a portion of verotoxin 1. Comparison between the particularity of NWGRI and that of NWGRL permits prediction that both Bcl-2 and Bcl-xL interact more strongly with verotoxin 2 than with verotoxin 1. In addition, the indexes obtained by the calculation from the result (Fig. 10) of the local alignment between verotoxin 1 and Bcl-2 or Bcl-xL are (27.0 + 26.0)/239 = 0.222 and 26.0/233 = 0.112, respectively (there is no homologous amino acid partial sequence other than the NWGR part). Consequently, it is predicted that the interaction of verotoxin 1 with Bcl-2 or Bcl-xL is considerably weaker than the interaction of verotoxin 2 with Bcl-2 or Bcl-xL.

## Example 5

Experimental confirmation of prediction of interaction of VTII with Bcl-2

In Example 4, the reliability of prediction that verotoxin 2 interacts with human Bcl-2 or Bcl-xL was predicted to be high. Based on the result of this prediction, it was experimentally confirmed that verotoxin 2 actually interacts with Bcl-2 (Fig. 11a and Fig. 11b (right)). Specifically, human hepatic cancer cell HepG2 (essentially not expressing the Bcl-2 gene) and B10 cells prepared by transducing a Bcl-2-expressing vector into HepG2 so as to express Bcl-2, were treated with verotoxin 2 (VTII), and then co-immunoprecipitation was conventionally carried out using anti-Bcl-2 antibody (Bcl-2 IPs) and anti-VTII antibody (VTII IPs).

Fig. 11a (left) illustrates the result of the western blotting analysis using anti-Bcl-2 antibody after co-immunoprecipitation; Fig. 11a (right) illustrates the result of the western blotting analysis using anti-VTII antibody. It was confirmed from these results that a VTII/Bcl-2 complex was co-immunoprecipitated in the B10 cells, i.e., these two proteins interact with each other. Moreover, B10 cells were treated with verotoxin 1 (VTI) or verotoxin 2 (VTII) to examine in which subcellular fraction these proteins were detected using anti-VTI antibody and anti-VTII antibody. Bcl-2 in mitochondria plays a very important role in cell death. Verotoxin 2 (VTII) was detected also in a mitochondria fraction (Fig. 11b (right)).

On the other hand, verotoxin 1 was not detected in the mitochondria fraction. Thus, it was proved experimentally that verotoxin 1 does not have a strong interaction with mitochondria Bcl-2. The result is shown in Fig. 11b (left).

## Example 6

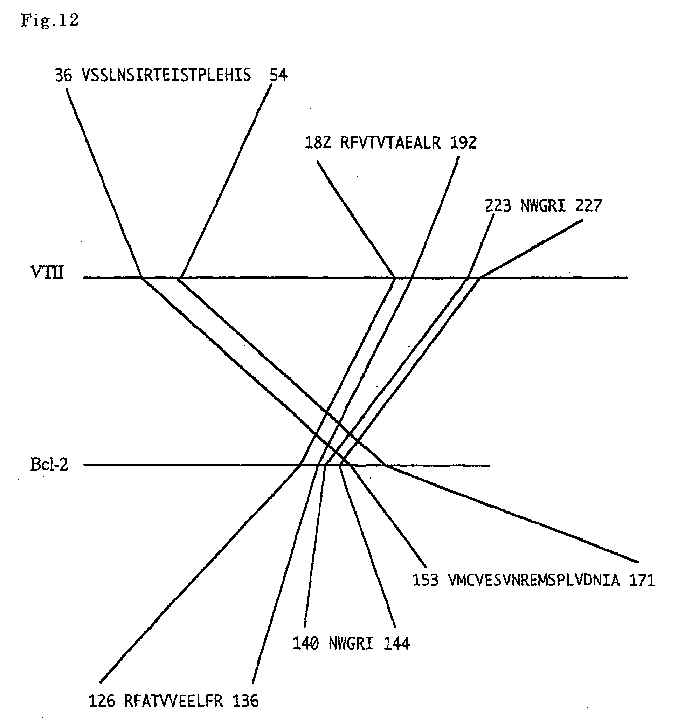

Fig. 12 illustrates an example wherein the full-length amino acid sequences of verotoxin 2 (VTII) and Bcl-2 were displayed so as to show the locations of the partial sequences aligned in the full-length sequences.

### Example 7

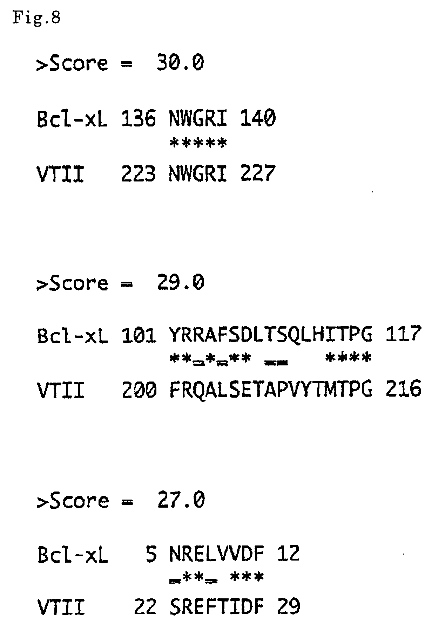

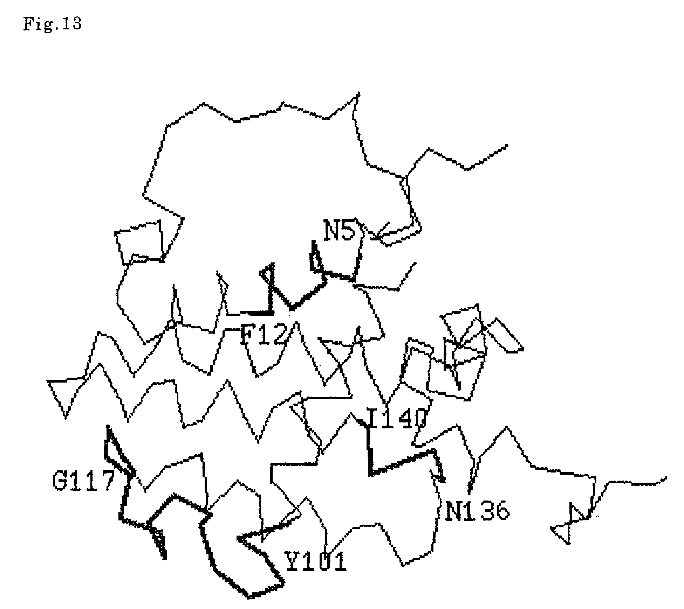

The stereo structure of Bcl-xL is known, with the structure being registered in PDB, that is a protein stereo structure database. Based on the result of the local alignment of Fig. 8, partial amino acid sequences homologous to those of verotoxin 2 in the stereo structure of Bcl-xL are shown with bold lines in Fig. 13.

### Example 8

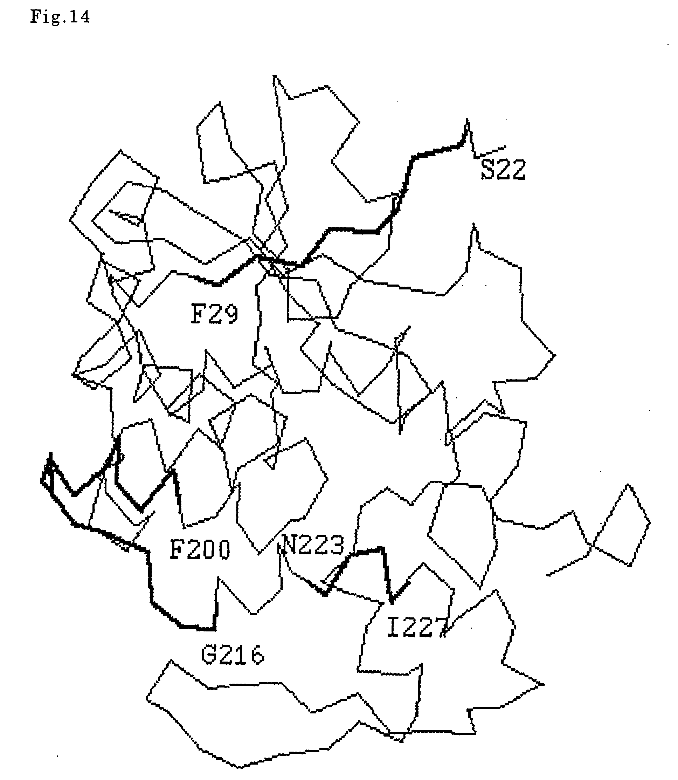

Verotoxin 2 is believed to cleave a part of ribosomal RNA so as to stop protein synthesis, thereby exerting its toxicity. The stereo structure of protein 'ricin' that cleaves a part of ribosomal RNA is registered in PDB, that is a protein stereo structure database. Based on the structure, homology modeling of verotoxin 2 was carried out. Based on the result of the local alignment of Fig. 8, the amino acid partial sequences homologous to those of Bcl-xL is shown in the stereo structure model with bold lines in Fig. 14.

### Example 9

Suppression by NWGRI of cell death induction by VTII

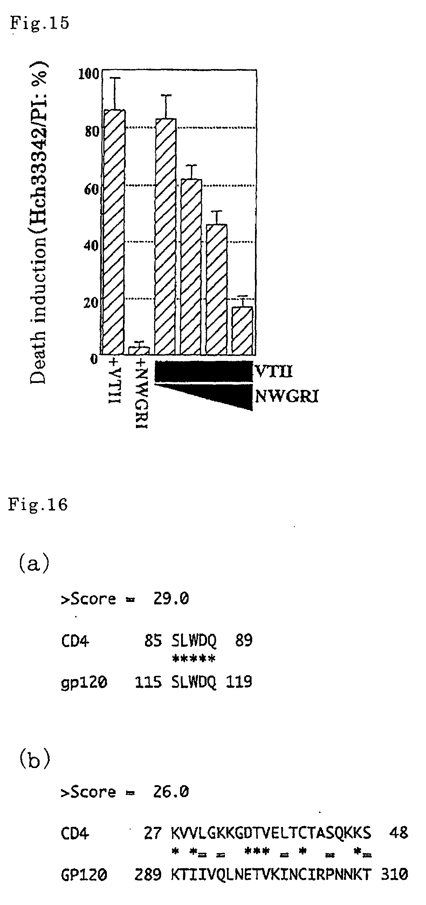

Next, it was experimentally confirmed that oligopeptide NWGRI (SEQ ID NO: 1), which was found in Example 4 and comprises a portion of VTII and Bcl-2, can control the interaction of VTII with Bcl-2. First of all, the complex formation was examined using an extract of the Bcl-2-expressing B10 cells used in Example 5 and biotinylated VTII in the presence of oligopeptide, and then analyzed by Far Western blotting analysis. Oligopeptide NWGRI interrupted the complex formation of VTII and Bcl-2 in a dose dependent manner.
In addition, B10 cells were pretreated with oligopeptide NWGRI at 0, 10, 50, 100 $\mu$M and were treated with VTII at 10 ng/ml for 24 hr, and the induction of cell death by apoptosis was assayed. A total of about 5,000 nuclei was dyed with Hoechst 33342/PI (Propidium iodide) according to the conventional method, and the ratio of nuclei that showed apoptosis is shown in Fig. 15. As shown in the figure, about 85% of cells caused apoptotic cell death by the treatment with only VTII, while the induction of apoptotic cell death was suppressed by pretreatment with oligopeptide NWGRI in a dose dependent manner. Thus, it was confirmed that oligopeptide NWGRI, which comprises a portion of VTII and Bcl-2, interrupts the interaction of VTII with Bcl-2 so as to inhibit the complex formation of these proteins and suppresses cell death induction by VTII thereupon.

### Example 10

CD4/gp120HIV-1

Human AIDS virus HIV-1 infects helper T cells. An important first step to infecting these cells is that protein gp120 on the viral surface binds to surface protein CD4 of helper T cells. In this example, it was examined if the binding of gp120 and CD4 can be predicted by the above prediction method.
Protein CD4 was decomposed into oligopeptides having an amino acid sequence length of 5 resides, and proteins having the amino acid sequence of the oligopeptide derived from CD4 were serially searched in a protein database, and gp120 was extracted as a protein that contains oligopeptide SLWDQ (Fig. 16a). Oligopeptide SLWDQ exists only in protein CD4 as a human protein in SWISS-PROT version 35, i.e., the frequency in human proteins is 1 and the particularity is very high. Moreover, besides this oligopeptide, a locally homologous region exists (Fig. 16b). It is known that amino acid residue arginine (Arg) next to oligopeptide SLWDQ in the N-terminal side and 67-SFLTKGP-73 play important roles when CD4 binds to gp120 (Kwong, PD., et al., Nature, vol. 398, 648-659, 1998). It is also known that a few amino acid residues next to the homologous region (289-KTIIVQLNETVKINCIRPNNKT-310) shown in Fig. 16b in the N-terminal side is one of the regions playing an important role when CD4 is recognized by gp120 (Kwong, PD., et al., Nature, vol. 398, 648-659, 1998). Therefore, even if the binding between gp120 and CD4 is not known, it can be predicted by the above prediction method.

### Example 11

CED-4/MAC-1

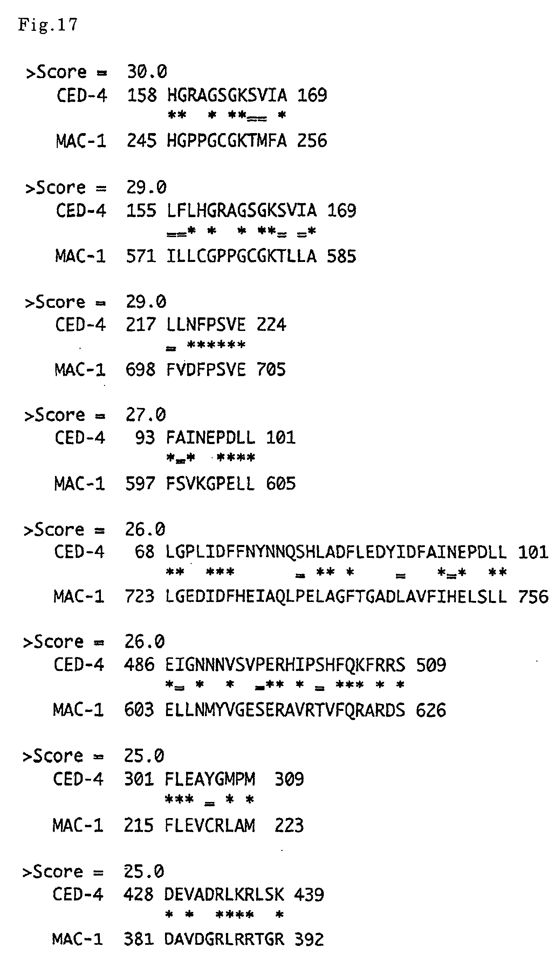

Nematode *Caenorhabditis elegans* is the first multicellular organism whose entire genome information was elucidated. One example concerning *C. elegans* is described here. Protein CED-4 plays a central role in the control of programmed cell death. MAC-1 was found to be a protein that binds to CED-4 and suppresses cell death (Wu et al., Development, vol. 126, 9, 2021-2031, 1999). Therefore, oligopeptides that comprise a portion of these two proteins were examined so as to verify the present invention, although the binding between MAC-1 and CED-4 is known. As a result, it was found that MAC-1 and CED-4 contain the same oligopeptide FPSVE having an amino acid sequence length of 5 residues, and the present invention was verified. The index of this oligopeptide, calculated from a frequency of amino acids in the genome of *C. elegans*, was 5.436. Moreover, as illustrated in Fig. 17, there are many homologous regions between these two proteins, whereby the binding of these proteins was strongly suggested (top sequence, CED-4; bottom sequence, MAC-1).

## Example 12

APP/BASE

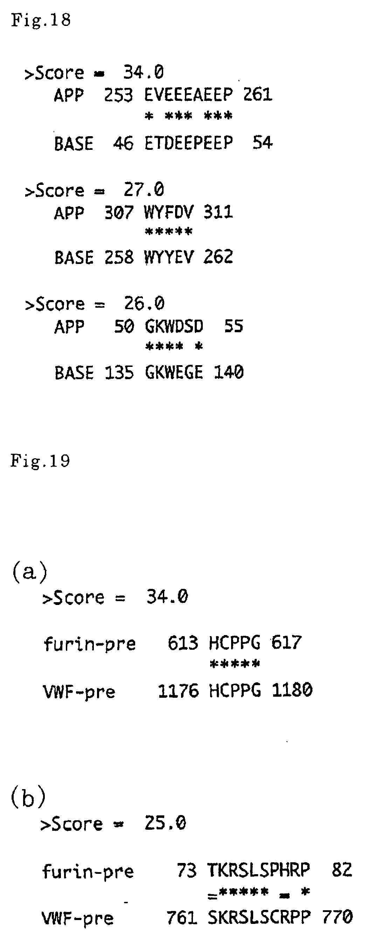

APP (amyloid precursor protein), which is one of the proteins causing Alzheimer's disease, gives rise to amyloid upon being cleaved at two sites. An enzyme (BASE, bata secretase) that cleaves the site on the amino terminal side of the two cleavage sites was recently discovered (VASSAR et al., Science, 286(5440), 735-741, 1999). Cleavage of APP by BASE indicates the presence of the interaction of these two proteins. To verify the present invention, oligopeptides that comprise a portion of these two proteins were examined. APP and BASE have homologous oligopeptides WYFDV and WYYEV having an amino acid sequence length of 5 residues that comprise a portion of each of them. The oligopeptide WYFDV exists only in protein APP as a human protein in SWISS-PROT version 35. A human protein comprising WYYEV is not registered yet. Both oligopeptides have high particularity. This result verified the present invention. Fig. 18 illustrates the regions homologous between the two proteins (top sequence, APP; bottom sequence, BASE).

## Example 13

Furin and von Willebrand factor

Furin is an intracellular serine protease, and is related to the secretion system pathway, such as von Willebrand factor (VWF), albumin, and complement C3. An example of the interaction of furin with VWF is mentioned here. VWF is cleaved from a precursor protein by furin to act as a mature protein. Cleavage of the VWF precursor protein by furin requires the interaction of these two proteins. Moreover, furin *per se* becomes a mature protein from a precursor protein of furin by being cleaved to act as a protease. Therefore, to verify the present invention, an oligopeptide that comprises a portion of furin precursor protein and VWF precursor protein was examined. The two proteins comprising the same oligopeptide HCPPG, at positions 613-617 of furin precursor protein and at positions 1176-1180 of VWF precursor protein (Fig. 19a). Both locations are within the regions of the mature proteins. The oligopeptide HCPPG comprises a portion of only furin precursor protein and VWF precursor protein as human proteins in SWISS-PROT version 35, and has high particularity from the viewpoint of frequency. VWF precursor protein is cleaved by furin at the site between the 763rd amino acid residue and the 764th amino acid residue. Local alignment between furin precursor protein and VWF precursor protein reveals that the region near the site of VWF precursor protein cleaved by furin has a partial region homologous to furin precursor protein (Fig. 19b). Thus, even if a novel protein was presumed to be a protease by the motif of the active part, and a counterpart protein as well as the cleavage site in the counter protein was not known, the present invention permits predicting the counterpart protein, as well as the cleavage site in the counter protein.

## Example 14

APP and PC7

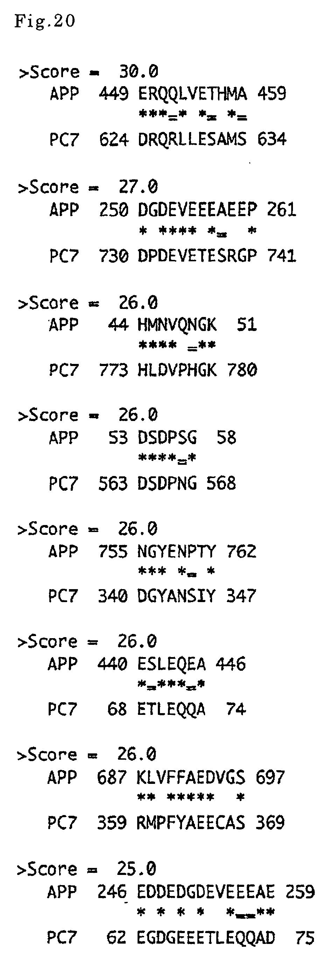

APP alpha is a peptide formed by cleavage of amyloid precursor protein (APP) at a site different from the two cleavage sites to form amyloid. It was recently found that PC7 (proprotein convertase subtilisin/kexin type 7) is involved in cleavage for forming APP alpha (Lopez-Perez E et al., J. Neurochem., vol. 73, 5, 2056-2062, 1999). Examination of the oligopeptide that comprises a portion of the two proteins APP and PC7 revealed that APP and PC7 have homologous oligopeptides DSDPSG and DSDPNG having an amino acid sequence length of 6 residues that comprise a portion of both of them. The oligopeptide DSDPSG exists only in protein APP as a human protein in SWISS-PROT version 35. A human protein comprising DSDPNG is not registered yet. Both oligopeptides have very high particularity. This result verified the present invention. Fig. 20 illustrates regions homologous between the two proteins.

Between K and L of 687-KLVFFAEDVGS-697 of APP in Fig. 20 is the cleavage site to form APP alpha, and Fig. 20 illustrates that a partial sequence (359-RMPFYAEECAS-369) homologous to this exists in PC7. Namely, this example shows that the present invention permits predicting a protein involved in cleaving another protein.

## INDUSTRIAL APPLICBILITY

As described above in detail, the present invention permits predicting, by using a protein database, a counterpart protein that interacts with a protein having an unknown function that is obtained by genome analysis or cDNA analysis or a protein having a known function. Namely, the protein-protein interaction in one organism whose genome information was elucidated can be predicted on a computer using a protein database based on genome analysis and cDNA analysis that have been recently enhanced. If the prediction on a computer becomes possible, information concerning proteins that were predicted to interact with each other based on the prediction on a computer can be easily obtained without adopting a risky technique wherein the result depends on a cDNA library used, such as the two-hybrid method. The prediction became possible, so that it becomes possible to easily predict the sequence of an oligopeptide involved in the interaction, and to design a novel compound capable of controlling protein-protein interactions based on the information. The present invention makes elucidating protein-protein interactions efficient, and can be widely utilized in various fields including biochemistry, molecular biology, pharmaceutical development, agriculture, and biotechnology. Especially, in the development of pharmaceuticals, the present invention permits predicting the mechanism of disease that has not so far been known, and gives a possibility of creating novel pharmaceuticals.

## Claims

1. A method for predicting a protein or polypeptide (B) that interacts with a specific protein or polypeptide (A), wherein the method is **characterized by** comprising:

    1) decomposing the amino acid sequence of protein or polypeptide (A) into a series of oligopeptides having a pre-determined length as sequence information;
    2) searching, within a database of protein or polypeptide amino acid sequences, for a protein or polypeptide (C) comprising an amino acid sequence for each member of the series or for a protein or polypeptide (D) comprising an amino acid sequence homologous to an amino acid sequence for each member of the series;
    3) carrying out local amino acid sequence alignment between said protein or polypeptide (A) and the detected protein or polypeptide (C) or detected protein or polypeptide (D); and
    4) predicting whether the detected protein or polypeptide (C) and/or protein or polypeptide (D) is a protein or polypeptide (B) that interacts with the protein or polypeptide (A) based on the results of the local amino acid sequence alignment and a value calculated from a frequency of amino acids and/or a frequency of said oligopeptides in said amino acid sequence database.

2. The method according to claim 1, wherein the oligopeptide is 4-15 amino acids in length.

3. A recording medium carrying a program to predict a protein or polypeptide (B) that interacts with a specific protein or polypeptide (A), wherein the recording medium is **characterized by** comprising at least the following means a) ~f):

> a) a means for inputting amino acid sequence information of the protein or polypeptide (A) and storing the information;
> b) a means for decomposing said information into a series of oligopeptides having a pre-determined length as sequence information, and a means for storing the sequence information consequently obtained;
> c) a means for storing an input protein database;
> d) a means for accessing the stored protein database and detecting a protein or polypeptide (C) having an amino acid sequence of said oligopeptide or a protein or polypeptide (D) having an amino acid sequence homologous to the amino acid sequence of said oligopeptide, and a means for storing and calculating a detected result;
> e) a means for carrying out local alignment between said protein or polypeptide (A) and the detected protein or polypeptide (C) or protein or polypeptide (D), and a means for storing and calculating a result; and
> f) a means for obtaining a resultant value of a frequency of an amino acid and/or a frequency of said oligopeptide from a protein database, followed by showing an index for predicting protein-protein interactions from the resultant value and a resultant value of said local alignment, and a means for storing and displaying the result and consequently detecting protein or polypeptide (B) which interacts with the protein or polypeptide (A).

4. A recording medium **characterized by** comprising at least the following means in addition to the means according to claim 3:

> g) a means for ranking strength of protein-protein interactions among detected proteins or polypeptides (B) based on the indexes calculated from a resultant value of local alignment and a resultant value of a frequency of an amino acid and/or a frequency of an oligopeptide in a protein database in the case that more than one protein or polypeptide (B) exist that are detected, and a means for storing and displaying the result.

5. A recording medium **characterized by** comprising at least the following means in addition to the means according to claim 3 or 4:

> h) a means for displaying full-length of amino acid sequences of said protein or polypeptide (A) and said protein or polypeptide (B) that is detected, followed by indicating a location of partial sequence to be aligned in the full-length sequence in the case that amino acid partial sequences are aligned by local alignment between the protein or polypeptide (A) and the protein or polypeptide (B).

6. A recording medium **characterized by** comprising at least the following means in addition to the means according to claim 3, 4 or 5:

> i) a means for calculating a stereo structure model in the case that a stereo structure of said protein or polypeptide (A) or said protein or polypeptide (B) that is detected is known or in the case that homology modeling enables to make a stereo structure model, followed by displaying the structure of the amino acid partial sequences that are aligned by local alignment between the protein or polypeptide (A) and the protein or polypeptide (B) on the stereo structure.

7. A recording medium **characterized by** comprising at least the following means in addition to the means according to claim 3, 4, 5 or 6:

> j) a means of classifying and storing proteins in a protein database to narrow a searching area.

8. A recording medium **characterized by** comprising at least the following means in addition to the means according to claim 3, 4, 5, 6 or 7:

k) a means for serially inputting each protein in a protein database as said protein or polypeptide (A).

9. A recording medium **characterized by** comprising at least the following means in addition to the means according to claim 3, 4, 5, 6, 7 or 8:

l) a means for storing a genome database.

10. A device for predicting protein-protein interactions which comprises the means that are carried by the recording medium according to claim 3, 4, 5, 6, 7, 8, or 9.

11. A method for specifying proteins or polypeptides that interact with each other, which comprises identifying a protein or polypeptide (B) that is predicted to interact with a specific protein or polypeptide (A) using the method according to claim 1 or 2, and then experimentally confirming the presence of the interaction between the protein or polypeptide (A) and the protein or polypeptide (B).

12. A method for specifying proteins or polypeptides that interact with each other, which comprises identifying a protein or polypeptide (B) that is predicted to interact with a specific protein or polypeptide (A) using the device according to claim 10, and then experimentally confirming the presence of the interaction between the protein or polypeptide (A) and the protein or polypeptide (B).

13. A protein or polypeptide that is specified by the method according to claim 11 or 12.

14. A method of screening for a compound that controls the interaction of a specific protein or polypeptide (A) with a protein or polypeptide (B), wherein the method utilizes the method according to claim 1 or 2.

15. A method of screening for a compound that controls the interaction of a specific protein or polypeptide (A) with a protein or polypeptide (B), wherein the method uses the device according to claim 10.

16. A novel compound obtained by the screening method according to claim 14 or 15.

17. A novel compound capable of controlling the interaction of a specific protein or polypeptide (A) with a protein or polypeptide (B), which is obtained by drug design based on information of a compound obtained by the screening method according to claim 14 or 15.

18. An oligopeptide comprising the amino acid sequence of SEQ ID No: 1, which is capable of controlling the interaction of verotoxin 2 (VTII) with Bcl-2.

19. An agent against cell death comprising an oligopeptide comprising the amino acid sequence of SEQ ID NO: 1.

20. An oligopeptide comprising an amino acid sequence homologous to the amino acid sequence of SEQ ID No: 1, which is capable of controlling the interaction of VTII with Bcl-2.

21. A polypeptide comprising the amino acid sequence of the oligopeptide according to claim 18 or 20, which is capable of controlling the interaction of VTII with Bcl-2.

22. A method of screening for a compound capable of controlling the interaction of VTII with Bcl-2, wherein the method utilizes the oligopeptide according to claim 18 or 20 and/or the polypeptide according to claim 21.

23. A method for determining the nucleotide sequence of an oligonucleotide coding an oligopeptide which is involved in the interaction of a specific protein or polypeptide (A) with a protein or polypeptide (B), wherein the method uses the prediction method according to claim 1 or 2 or the prediction device according to claim 10.

24. A series of combinations of human proteins that are predicted to interact with each other, obtained by the method according to claim 1 or 2 or the device according to claim 10.

25. A method for selecting a combination of proteins which interact with each other, wherein said interaction is related to a disease, wherein the method comprises selecting the combination based on the information of a known protein that can be related to the disease from the series of combinations according to claim 24.

26. A series of combinations of proteins which interact with each other, wherein said interaction is related to a disease, wherein each member of said series is selected according to the method of claim 25.

27. A method of screening for a compound which controls the interaction of a combination of proteins and/or two proteins selected from the series of combinations according to claim 26.

28. A compound obtained by the method according to claim 27.

29. A method for predicting a processing site of a protein comprising predicting the interaction of a specific protein with an enzyme cleaving said protein using the method according to claim 1 or 2 or the device according to claim 10.

30. A polypeptide comprising an amino acid sequence that is predicted to contain a protein-processing site obtained by the method according to claim 29 and/or to contain a partial sequence homologous to the processing site.

Fig.1

(a)

MKCILFKWVLCLLLGFSSVS

(b)

MKCIL
KCILF
CILFK
ILFKW
LFKWV
FKWVL
KWVLC
WVLCL
VLCLL
LCLLL
CLLLG
LLLGF
LLGFS
LGFSS
GFSSV
FSSVS

(c)

MKCILF
KCILFK
CILFKW
ILFKWV
LFKWVL
FKWVLC
KWVLCL
WVLCLL
VLCLLL
LCLLLG
CLLLGF
LLLGFS
LLGFSS
LGFSSV
GFSSVS

Fig.2

MKCIL
  none

KCILF
  BETA-ADRENERGIC RECEPTOR KINASE 2 (ARK2_HUMAN)

CILFK
  WHITE PROTEIN HOMOLOG (WHIT_HUMAN)

ILFKW
  none

LFKWV
  none

FKWVL
  none

KWVLC
  none

WVLCL
  none

VLCLL
  GROWTH ARREST AND DNA-DAMAGE-INDUCIBLE PROTEIN GADD45
  (GA45_HUMAN)

Fig.3

>Score = 32.0

    ARK2  618 KCILFR 623
               ******
    VTII    2 KCILFK 7

>Score = 27.0

    ARK2  651 FKEAQRLLRRA 661
              **= ** =*=*
    VTII  193 FRQIQREFRQA 203

>Score = 26.0

    ARK2  299 TEIILGLEHV 308
              *** ****
    VTII   44 TEISTPLEHI 53

>Score = 26.0

    ARK2  379 CMLFK 383
               *****
    VTII    3 CILFK 7

>Score = 25.0

    ARK2   12 SYLMAMEKSKATPAARASKRI 32
               *** ** * * = *** *
    VTII  160 SYLALMEFSGNTMTRDASRAV 180

Fig. 4

## (a)                    (b)

| | |
|---|---|
| L: 144615 | L: 10.639652% |
| A: 128924 | A: 9.485230% |
| G: 100162 | G: 7.369144% |
| V: 95941 | V: 7.058596% |
| I: 81611 | I: 6.004305% |
| S: 79179 | S: 5.825378% |
| E: 78102 | E: 5.746140% |
| R: 75293 | R: 5.539476% |
| T: 73489 | T: 5.406752% |
| D: 69829 | D: 5.137477% |
| Q: 60214 | Q: 4.430080% |
| P: 60182 | P: 4.427726% |
| K: 59895 | K: 4.406610% |
| N: 53727 | N: 3.952817% |
| F: 52995 | F: 3.898962% |
| M: 38745 | M: 2.850557% |
| Y: 38721 | Y: 2.848791% |
| H: 30912 | H: 2.274266% |
| W: 20761 | W: 1.527434% |
| C: 15911 | C: 1.170608% |

Fig.5

Fig.6

```
LCLLL      FASA_HUMAN
QRVAA      TRAI_HUMAN
EFSGN      DAPK_HUMAN
NWGRI      BCL2(BC2A)_HUMAN, BCLX_HUMAN, MCL1_HUMAN
```

Fig.7

```
>Score =  30.0

Bcl-2  143 NWGRI 147
           *****
VTII   223 NWGRI 227



>Score =  27.0

Bcl-2  129 RFATVVEELFR 139
           ** **  * _*
VTII   182 RFVTVTAEALR 192



>Score =  25.0

Bcl-2  156 VMCVESVNREMSPLVDNIA 174
           *    **  ***    *_*_
VTII    36 VSSLNSIRTEISTPLEHIS  54
```

Fig.8

>Score =  30.0

Bcl-xL 136 NWGRI 140
                *****
VTII    223 NWGRI 227


>Score =  29.0

Bcl-xL 101 YRRAFSDLTSQLHITPG 117
                **⌐*⌐**  ⬛⬛    ****
VTII    200 FRQALSETAPVYTMTPG 216


>Score =  27.0

Bcl-xL   5 NRELVVDF 12
                ⬛**⬛ ***
VTII    22 SREFTIDF 29

Fig.9

>Score = 34.0

Mcl-1  326 GGIRNVLLAFAGVAGVGAG 344
               * * *** * *  ** *
VTII   225 GRISNVLPEYRGEDGVRVG 243


>Score = 30.0

Mcl-1  260 NWGRI 264
               *****
VTII   223 NWGRI 227


>Score = 28.0

Mcl-1  275 AKHLKTINQES 285
               **  *=**=**
VTII   268 ARSVRAVNEES 278


>Score = 26.0

Mcl-1  134 LGKRPAVLPLLELVGESGNNTSTDGS 159
               = *== * = * *= *** = * *
VTII   152 ISRHSLVSSYLALMEFSGNTMTRDAS 177

Fig.10

(a)

>Score = 27.0

Bcl-2 129 RFATVVEELFR 139
             ** ** * _*
VTI   182 RFVTVTAEALR 192


>Score = 26.0

Bcl-2  143 NWGRI 147
               ****_
VTI    224 NWGRL 228


(b)

>Score = 26.0

Bcl-xL   136 NWGRI 140
               ****_
VTI      224 NWGRL 228

Fig.11

(a)

Bcl-2 WB

Bcl-2 IPs    VTII IPs

HepG2  B10  HepG2  B10

VTII WB

Bcl-2 IPs    VTII IPs

HepG2  B10  HepG2  B10

(b)

VTII  Nuclear  Cytosol  Membrane  Mitochondria

VTII  Nuclear  Cytosol  Membrane  Mitochondria

Fig.12

36 VSSLNSIRTEISTPLEHIS 54

182 RFVTVTAEALR 192

223 NWGRI 227

VIII

Bcl-2

153 VMCVESVNREMSPLVDNIA 171

140 NWGRI 144

126 RFATVVEELFR 136

Fig.13

Fig.14

Fig.15

Fig.16

(a)

>Score = 29.0

CD4      85 SLWDQ  89
            *****
gp120   115 SLWDQ 119

(b)

>Score = 26.0

CD4      27 KVVLGKKGDTVELTCTASQKKS  48
            *  *= =   *** = *  =   *=
GP120   289 KTIIVQLNETVKINCIRPNNKT 310

Fig.17

```
>Score =  30.0
   CED-4  158 HGRAGSGKSVIA 169
               **   * ** = *
   MAC-1  245 HGPPGCGKTMFA 256


>Score =  29.0
   CED-4  155 LFLHGRAGSGKSVIA 169
               ==* *   * **= =*
   MAC-1  571 ILLCGPPGCGKTLLA 585


>Score =  29.0
   CED-4  217 LLNFPSVE 224
               = ******
   MAC-1  698 FVDFPSVE 705


>Score =  27.0
   CED-4   93 FAINEPDLL 101
               *=*  ****
   MAC-1  597 FSVKGPELL 605


>Score =  26.0
   CED-4   68 LGPLIDFFNYNNQSHLADFLEDYIDFAINEPDLL 101
               **   ***        = ** *    =    *=*  **
   MAC-1  723 LGEDIDFHEIAQLPELAGFTGADLAVFIHELSLL 756


>Score =  26.0
   CED-4  486 EIGNNNVSVPERHIPSHFQKFRRS 509
               *= *   *   =** * = *** * *
   MAC-1  603 ELLNMYVGESERAVRTVFQRARDS 626


>Score =  25.0
   CED-4  301 FLEAYGMPM  309
               *** = * *
   MAC-1  215 FLEVCRLAM  223


>Score =  25.0
   CED-4  428 DEVADRLKRLSK 439
               * *  ****  *
   MAC-1  381 DAVDGRLRRTGR 392
```

Fig.18

```
>Score = 34.0
   APP  253 EVEEEAEEP 261
            * *** ***
   BASE  46 ETDEEPEEP  54


>Score = 27.0
   APP  307 WYFDV 311
            *****
   BASE 258 WYYEV 262


>Score = 26.0
   APP   50 GKWDSD  55
            **** *
   BASE 135 GKWEGE 140
```

Fig.19

(a)
```
   >Score = 34.0

   furin-pre    613 HCPPG 617
                    *****
   VWF-pre     1176 HCPPG 1180
```

(b)
```
   >Score = 25.0

   furin-pre     73 TKRSLSPHRP  82
                    =***** = *
   VWF-pre      761 SKRSLSCRPP 770
```

Fig.20

```
>Score =  30.0
   APP  449 ERQQLVETHMA 459
             ***_* *_ *_
   PC7  624 DRQRLLESAMS 634


>Score =  27.0
   APP  250 DGDEVEEEAEEP 261
             * **** *_  *
   PC7  730 DPDEVETESRGP 741


>Score =  26.0
   APP   44 HMNVQNGK  51
             **** _**
   PC7  773 HLDVPHGK 780


>Score =  26.0
   APP   53 DSDPSG  58
             ****_*
   PC7  563 DSDPNG 568


>Score =  26.0
   APP  755 NGYENPTY 762
             *** *_ *
   PC7  340 DGYANSIY 347


>Score =  26.0
   APP  440 ESLEQEA 446
             *_***_*
   PC7   68 ETLEQQA  74


>Score =  26.0
   APP  687 KLVFFAEDVGS 697
             ** ***** *
   PC7  359 RMPFYAEECAS 369


>Score =  25.0
   APP  246 EDDEDGDEVEEEAE 259
             * * * * *_ _**
   PC7   62 EGDGEEETLEQQAD 75
```

## INTERNATIONAL SEARCH REPORT

| | International application No. |
|---|---|
| | PCT/JP01/01846 |

**A. CLASSIFICATION OF SUBJECT MATTER**
Int.Cl$^7$  G06F17/30, C07K7/06, C07K14/435, G01N33/68

According to International Patent Classification (IPC) or to both national classification and IPC

**B. FIELDS SEARCHED**

Minimum documentation searched (classification system followed by classification symbols)
Int.Cl$^7$  G06F17/30, C07K7/06, C07K14/435, G01N33/68

Documentation searched other than minimum documentation to the extent that such documents are included in the fields searched
Jitsuyo Shinan Koho        1922-1996    Toroku Jitsuyo Shinan Koho  1994-2001
Kokai Jitsuyo Shinan Koho  1971-2001    Jitsuyo Shinan Toroku Koho  1996-2001

Electronic data base consulted during the international search (name of data base and, where practicable, search terms used)
JICST FILE(JOIS)(verotoxin, BCL, protein, interaction) (in Japanese)
INSPEC(DIALOG)
REGISTRY(STN),CA(STN),MEDLINE(STN)

**C. DOCUMENTS CONSIDERED TO BE RELEVANT**

| Category* | Citation of document, with indication, where appropriate, of the relevant passages | Relevant to claim No. |
|---|---|---|
| A | MARCOTTE, E. M. et al., "Detecting Protein Function and Protein-Protein Interactions from Genome Sequences" Science, Vol.285, No.5428, 30 July 1999 (30.07.99) pp.751-753 | 1-12,14,15, 23,29 |
| PX | SUZUKI, A. et al., "Bcl-2 antiapoptotic protein mediates verotoxin II-induced cell death: possible association between cl-2 and tissue failure by E. coli O157:H7" GENES & DEVELOPMENT, Vol.14, No.14, 15 July 2000 (15.07.00) pp.1734-1740 | 1-12,14,15, 18-23 |

☐ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 04 June, 2001 (04.06.01) | 12 June, 2001 (12.06.01) |

| Name and mailing address of the ISA/ | Authorized officer |
|---|---|
| Japanese Patent Office | |
| Facsimile No. | Telephone No. |

Form PCT/ISA/210 (second sheet) (July 1992)

## INTERNATIONAL SEARCH REPORT

| International application No. |
| --- |
| PCT/JP01/01846 |

**Box I**  **Observations where certain claims were found unsearchable (Continuation of item 1 of first sheet)**

This international search report has not been established in respect of certain claims under Article 17(2)(a) for the following reasons:

1. ☐ Claims Nos.:
   because they relate to subject matter not required to be searched by this Authority, namely:

2. ☒ Claims Nos.: 13,16,17,24-28,30
   because they relate to parts of the international application that do not comply with the prescribed requirements to such an extent that no meaningful international search can be carried out, specifically:

   It is unknown what particular compounds are involved and what compounds are not in the scopes of "protein", "polypeptide", "novel compound" and "a group of combinations of proteins" as stated in the above claims. Thus, these claims are described in an extremely unclear manner. Therefore, no meaningful international search can be practiced on these claims.

3. ☐ Claims Nos.:
   because they are dependent claims and are not drafted in accordance with the second and third sentences of Rule 6.4(a).

**Box II**  **Observations where unity of invention is lacking (Continuation of item 2 of first sheet)**

This International Searching Authority found multiple inventions in this international application, as follows:

   The inventions as set forth in claims 1 to 17 and 23 relate to the computed anticipation of an interaction between proteins.
   The inventions as set forth in claims 18 to 22 relate to the interaction between Vero toxin and Bcl-2.
   The inventions as set forth in claims 24 to 28 relate to the interaction between human-origin proteins participating in diseases.
   The inventions as set forth in claims 29 and 30 relate to the interaction between a protein and a cleaving enzyme.
   These groups of inventions can be considered neither as a single group of inventions nor a group of inventions so linked as to form a single general inventive concept.

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**  ☐  The additional search fees were accompanied by the applicant's protest.
   ☒  No protest accompanied the payment of additional search fees.

Form PCT/ISA/210 (continuation of first sheet (1)) (July 1992)